# EUROPÄISCHE PATENTSCHRIFT

(11) **EP 1 085 919 B1**
(45) Veröffentlichungstag und Bekanntmachung des Hinweises auf die Patenterteilung: **17.10.2001**
(21) Anmeldenummer: 99941393.3
(22) Anmeldetag: 24.06.1999
(51) Int. Cl.: A61L 27/00

(54) **AUTOLOGE EPITHELIALISIERUNG BZW. ENDOTHELIALISIERUNG VON HOHLORGANEN BZW. GEFÄSSEN**
AUTOLOGOUS EPITHELIALISATION OR ENDOTHELIALISATION OF HOLLOW ORGANS OR VESSELS
EPITHELIALISATION OU ENDOTHELIALISATION AUTOLOGUE D'ORGANES CREUX OU DE VAISSEAUX

(30) Priorität: 25.06.1998 DE 19828428; 09.09.1998 DE 19841264
(43) Veröffentlichungstag der Anmeldung: 28.03.2001
(73) Patentinhaber: Vascular Biotech GmbH, 80331 München (DE)
(72) Erfinder: NEES, Stephan, D-81375 München (DE); LAMM, Wilhelm, Peter, D-82256 Fürstenfeldbruck (DE)
(74) Vertreter: Grund, Martin, Dr.
(86) Internationale Anmeldenummer: DE9901871
(87) Internationale Veröffentlichungsnummer: WO9966965

(56) Entgegenhaltungen:
- WO-A-93/01843
- WO-A-95/31944
- US-A- 5 246 451

## Beschreibung

Die Erfindung betrifft natürliche Kryopräservierte Hohlorgane und deren sämtliche Bestandteile, insbesondere Gefäße und ihre Klappen, die an der Innenoberfläche bzw. der luminalen Oberfläche eine Auskleidung mit Patienten-autologem Epithel, insbesondere Endothelzellen, aufweisen, ein Verfahren zur Herstellung dieser Hohlorgane und die Verwendung dieser Hohlorgane zur Herstellung eine Arzneimittels zur Verwendung in der Chirurgie, insbesondere in der Herz- und Gefäßchirurgie.

Die Kryopräservation und Lagerung von Organen und Körpergeweben zur Konservierung und späteren Verwendung z.B. im Rahmen einer Transplantation sind bekannt und weitgehend standardisiert. Die verwendeten Techniken unterscheiden sich dabei nur unwesentlich (Brockbank KGM. Basic Principles of Viable Tissue Preservation. In: Transplantation Techniques and Use of Cryopreserved Allograft Cardiac Valves and Vascular Tissue. DR Clarke (ed.), Adams Publishing Group Ltd., Boston. S 9-23. American Association of Tissue Banks Standards for Tissue Banking (1995), A.A.T.B., McLean, VA, U.S.A. European Association of Tissue Banks General Standards for Tissue Banking (1995), E.A.T.B., Vienna, Austria).
Der Einsatz von kryopräservierten Venenallografts ist ein etabliertes Verfahren in der Bypasschirurgie (Brockbank KGM et al., Cryopreserved vein transplantation. J. Cardiac Surg. 7:170-176, 1992; Gelbfish J. et al., Cryopreserved homologous saphenous vein: Early and late patency in coronary artery bypass surgical procedures. Ann. Thorac. Surg. 42:70, 1986; Fujitani RM et al., Cryoperserved saphenous vein allogenic homografts: An alternative conduit in lower extremity arterial reconstruction in infected fields. J. Vasc. Surg. 15: 519-526, 1992) und findet bei den Patienten Verwendung, die nicht über genügend körpereigenes Gefäßmaterial verfügen, oder deren Gefäße qualitativ nicht geeignet sind. Derartig eingesetzte Venen zeigen einen schlechten Langzeitverlauf (Bilfinger TV et al., Cryopreserved Veins in Myocardial Revascularization: Possible Mechanism for Their Increased Failure. Ann. Thorac. Surg. 63: 1063-69, 1997 und Kommentar Ann. Thorac. Surg. 64: 1524-5, 1997. Marshin RS et al., Cryopreserved Saphenous Vein Allografts for Below Knee Lower Extremity Revascularization. Ann. Surg. 219: 664-72, 1994). Ursächlich hierfür dürfte vor allem eine immunologisch bedingte Degeneration sein (Carpenter JP, Tomaszewski JE, Immunosuppression for Human Saphenous Vein Allograft Bypass Surgery: a Prospective Randomized Trial. J. Vasc. Surg. 26: 32-42, 1997. Carpenter JP, Tomaszewski JE, Human Saphenous Vein Allograft Bypass Grafts: Immune Response. J. Vasc. Surg. 27:492-9, 1998). Darüber hinaus werden häufig thrombotische Frühverschlüsse beobachtet. Beide Prozesse wurden bislang auf die Beschädigung des Spenderendothels im Rahmen der Kryopräservierung, die bis zum vollständigen Fehlen desselben führen kann, oder auf eine eingeschränkte Funktionalität des erhaltenen Endothels zurückgeführt (Brockbank KGM et al., Cryopreserved vein transplantation. J. Cardiac Surg. 7:170-176, 1992; Brockbank KGM et al., Functional analysis of cyropreserved veins. J. Vasc. Surg. 11: 94-102, 1990. Laub GW et al., Cryopreserved allograft veins as alternative coronary conduits: early phase results. Ann. Thorac. Surg. 54: 826-31,1992. Louagie YA et al., Viability of long term cryopreserved human saphenous veins. J. Cardiovasc. Surg. 31: 92-100, 1990). Demzufolge zielen bislang bekannte und bereits patentierte Kryopräservationstechniken für Allo- und Xenografts darauf ab, einen möglichst hohen Erhaltungsgrad des vaskulären und mikrovaskulären Spenderendothels nach dem Reinigungsprozeß zu gewährleisten. Der Erhaltungsgrad des Spenderendothels des kryopräservierten Gewebes wird in der Literatur mit 50% - 80% angegeben (Bambang LS et al., Effects of cryopreservation on the proliferation and anticoagulant activity of human saphenous vein endothelial cells. J. Thorac Cardiovasc. Surg. 110: 998-1004).

In neuerer Zeit wird jedoch gerade dem vaskulären und mikrovaskulären Endothel im Rahmen der akuten und chronischen Organabstoßung eine Schlüsselposition beigemessen. Endothelspezifische Nicht-HLA-Antigene, die zu einer Aktivierung von CD4 T-Zellen führen, ermöglichen es dem Spenderendothel - im Zusammenwirken mit weiteren akzessorischen Molekülen - fremde Antigene dem Immunsystem des Empfängers anzubieten. Die Freisetzung von Nicht-HLA-Antigenen durch beschädigte Endothelzellen führt zu einer chronischen Immunreaktion und möglicherweise zur Graftvaskulopathie und chronischen Abstoßung (Rose ML, Role of endothelial cells in allograft rejection. Vasc. Med. 2(2): 105-14, 1997; Reul RM, Fang JC, Denton MD et al, CD 40 and CD 40 ligand (CD 154) are coexpressed in microvessels in vivo in human cardiac allograft rejection. Transpalantation 64(12): 1765-74, 1997; Salom RN, Maguire JA, Hancock WW, Endothelial activation and cytokine expression in human acute cardiac allograft rejection. Pathology 30(1): 24-29, 1998). In den US-Patenten No. 5,843,182 und No. 5,613,982 wird ein Verfahren zur Herstellung einer nicht-immunogenen Gewebematrix vorgeschlagen. Bei diesem Verfahren wird zunächst eine Dezellularisierung zur Entfernung der Spenderzellen mit Hilfe hydrolytischer Enzyme (z.B. Proteasen, Lipasen, Nukleotidasen, Glykosidasen etc.) durchgeführt und nachfolgend die gewonnene Matrix mit zellulären Adhäsionsfaktoren bzw. Wachstumsfaktoren behandelt, um eine Ansiedlung von Fibroblasten zu ermöglichen.

Derartige Maßnahmen haben aber den prinzipiellen Nachteil, daß nie ausgeschlossen werden kann, daß diese Behandlungen auch die Festigkeit anderer, für die strukturelle Integrität von Hohlorganwänden ebenfalls entscheidender Komponenten wie z.B. der Kollagene, insbesondere Typ I Kollagen, Proteoglykane oder Glykoproteine schädigend beeinflussen.

Dies ist um so wichtiger, da schwerste Komplikationen, die auf einer strukturellen Wandschwäche der kryopräservierten Venen beruhen, wie z.B. Einrisse der Venenwand oder Wandaussackungen (Aneurysmen) der Venen, die zu komplikationsträchtigen Reoperationen führen, auch längere Zeit nach der Implantation in den Menschen bestens bekannt sind (Lehalle B et al., Early rupture and degeneration of cryopreserved arterial allografts. J. Vasc. Surg. 25: 751-2, 1997. Couvelard A. et al., Human allograft failure. Hum. Pathol. 26: 1313-20, 1995). Wenn dezellularisiertes Gewebe als Matrix für Rezellularisierungsmaßnahmen dient, wird es notwendig, das zu transplantierende Gewebe mit hohen Konzentrationen spezieller Adhäsionsfaktoren bzw. Wachstumsfaktoren zu inkubieren, um eine erneute Ansiedlung von Zellen in der Wand zu ermöglichen (U.S. Pat. No. 5,632,778 und 5,613,982 bzw. U.S. Pat. No. 5,192,312, U.S. Pat. No. 5,843,182, WO 95/24873). Derartige Präparate sind teuer, meist nicht für den klinischen Gebrauch zugelassen und es ist noch weitgehend unbekannt, in welchem Ausmaß unphysiologisch hohe Konzentrationen dieser Wirkstoffe die funktionelle Differenzierung von Geweben beeinflussen.

Eine Vorbehandlung der Matrix, die zu einer Dezellularisierung führt, weist ebenso wie eine Behandlung der dezellularisierten Matrix mit Adhäsionsfaktoren bzw. Wachstumsfaktoren nicht unerhebliche zellbiologische und immunologische Risiken auf.

Außer diesen Nachteilen werden in der betreffenden Literatur bisher kaum die Erkenntnisse der Literatur zur Verteilung antithrombogener bzw. prothrombogener Aktivitäten bzw. Wirkstrukturen in der Wand von Hohlorganen berücksichtigt. Während sich vaskuläres Endothel (als Deckgewebe der Innen- bzw. luminalen Seite von allen Blutgefäßen und Blutgefäßklappen) z.B. durch eine Vielzahl antiaggregatorischer, antikoagulatorischer und profibrinolytischer Aktivitäten auszeichnet (Z. Kardiol. 82: Suppl. 5, 13-21, 1993; FASEB J. 2: 116-123, 1988), sind zelluläre Komponenten der tieferen Gefäßwand vor allem durch die Expression von Gewebefaktor charakterisiert, der im Kontakt mit Plasmafaktoren eine sofortige Gerinnungsreaktion einleitet (Thrombosis Res. 81: 1-41, 1996; J. Clin. Invest. 100: 2276-2285, 1997; FASEB J. 8: 385-390, 1994; Arterioscler. Thromb. Vasc. Biol. 17: 1-9, 1997). Eine Abschirmung gerade der prothrombogenen Wandaktivitäten von Hohlorganen ist nicht nur bei Blutgefäßen und Blutgefäßklappen, sondern auch bei allen anderen kryopräservierten und nicht kryopräservierten natürlichen oder künstlichen Hohlorganen bzw. Gefäßen von größter physiologischer Wichtigkeit

Der Erfindung lag daher die Aufgabe zugrunde, verbessertes Gefäßmaterial für die Herz- und Gefäßchirurgie bereitzustellen.

Eine weitere Aufgabe der vorliegenden Erfindung besteht ferner darin, ein geeignetes Verfahren zur Herstellung von derartigem Gefäßmaterial, insbesondere von Blutgefäßen und deren Klappen, für chirurgische Transplantationszwecke bereitzustellen.

Diese Aufgabe wird erfindungsgemäß gelöst durch natürliche kryopräservierte Hohlorgane und deren sämtliche Bestandteile, die an der Innenoberfläche bzw. der luminalen Oberfläche eine Auskleidung mit Empfänger (=Patienten)-autologem Epithel aufweisen. Bevorzugte erfindungsgemäße Hohlorgane weisen an der Innenoberfläche bzw. der luminalen Oberfläche eine Auskleidung mit Patienten-autologen Endothelzellen auf. Eine besonders bevorzugte Ausführungsform der vorliegenden Erfindung umfaßt Gefäße und ihre Klappen, die mit Empfänger-autologen Endothelzellen auf der Innenoberfläche bzw. der luminalen Oberfläche ausgekleidet sind.

Die erfindungsgemäßen Hohlorgane, insbesondere die erfindungsgemäßen Gefäße, die mit Patienten-antologen Endothelzellen auf der Innenoberfläche ausgekleidet sind, weisen eine bessere Langzeitoffenheitsrate als nicht beschichtete Hohlorgane bzw. Gefäße auf.

Beispiele für erfindungsgemäße Hohlorgane, die an der Innenoberfläche bzw. der luminalen Oberfläche eine Auskleidung mit Patienten-autologem Epithel aufweisen, sind natürliche Blutgefäße und ihre Klappen, Lymphgefäße und ihre Klappen, Harnleiter und Harnblase, Samenleiter, Bronchien, das Herz als spezielles Blutgefäß mit seinen Klappen und jeglicher prosthetischer Ersatz solcher Hohlorgane.

Als besonders bevorzugte erfindungsgemäße Gefäße kommen kryopräservierte allo- oder xenogene Gefäße (Arterien, Venen, Lymphgefäße), die mit autologen Endothelzellen auf der Innenoberfläche ausgekleidet sind, in Betracht

Ein weiterer Vorteil der Erfindung ist, daß die epitheliale Auskleidung (Beschichtung) der erfindungsgemäßen Hohlorgane konfluent und dauerhaft verankert ist.

Dies ist besonderes bedeutsam, da das Endothel aller größeren Blutgefäße, vaskulären Klappen und Herzhöhlen im gesunden, intakten Zustand gleichzeitig als anatomische, physikalische und metabolische Barriere zwischen dem Blut und den tieferen Wandschichten der genannten vaskulären Strukturen fungiert und so erst eine eigenständige, getrennte Regulation zahlreicher physiologischer Prozesse in diesen beiden Körperkompartimenten zuläßt.

Insbesondere wird durch die erfindungsgemäßen zu transplantierenden Blutgefäße, Klappen und Herzhöhlen, die mit Patienten-autologen Endothelzellen auf der luminalen Oberfläche ausgekleidet sind, nicht nur die Entstehung von Thromboembolien verhindert und keine Immunantwort an ihrer luminalen Oberfläche ausgelöst, sondern es wird auch eine akute Infiltration der tieferen Wandschichten mit unspezifisch wirkenden Abwehrzellen (Granulozyten, Monozyten) verhindert. Wie die gesammelte eigene klinische Erfahrung bereits zeigt, kommt es aus diesem Grund nicht zu einer klinisch relevanten Abstoßungsreaktion gegenüber transplantierten, autolog endothelialisierten Blutgefäßen.

Weiterhin betrifft die Erfindung ein Verfahren zur Herstellung, der erfindungsgemäßen Hohlorgane. Das erfindungsgemäße Beschichtungsverfahren umfaßt die Beschichtung, insbesondere die dauerhafte Beschichtung, von einem kyropräservierten Spenderhohlorgan mit Patienten-autologem Epithel, insbesondere die Beschichtung von einem kyropräservierten oder nicht kyropräservierten Spendergefäß mit Patienten-autologen Endothelzellen.

In einer bevorzugten Ausführungsform umfaßt das erfindungsgemäße Beschichtungsverfahren zusätzlich die Vorbeschichtung der kyropräservierten Gefäße mit Patienten-autologem Serum.

In einer besonders bevorzugten Ausführungsform wird die Beschichtung eines Gefäßes (bzw. eines Hohlorgans) mit Patienten-autologen Endothelzellen mit Hufe eines speziellen Kultivationsgerätes durchgeführt, das dadurch gekennzeichnet ist, daß ein Druckgradient aufgebaut werden kann zwischen dem Lumen des Gefäßes (bzw. Hohlorgans) und dem Außenbereich des Gefäßes, der verhindert, daß das die Wände des Gefäßes (bzw. des Hohlorgans) kolabieren.

Die auf diese Weise hergestellten erfindungsgemäßen Hohlorgane weisen erhebliche Vorteile auf. Es gelang auf der luminalen Oberfläche, z.B. eines Blutgefäßes, eine absolut konfluente, gegen die Scherkräfte des vorbeiströmenden Blutes dauerhaft verankerte Endothelschicht zu etablieren. Diese wirkt nicht nur als komplexer antithrombogener Katalysator, um die Thromboembolisierung der Hohlorgane zu verhindern, sondern auch als wirksamer Schutz gegen eine für die deletäre Abstoßung der tieferen Wandstrukturen des Hohlorgans im Sinne einer akuten Entzündungsreaktion unabdingbare, massenhafte Rektrutierung von Abwehrzellen aus dem Blut.

Das erfindungsgemäße Verfahren führt zur dauerhaften Reendothelialisierung mit Patientenautologem Endothel des Empfängers.

In einer bevorzugten Ausführungsform wird eine möglichst selektive De-Epithelialisierung des kyropräservierten Spenderhohlorgans (= Entfernung des Spenderepithels) vor der Beschichtung mit Patienten-autologen Epithelzellen (=Aufbringen von Empfängerepithel) durchgeführt.

Insbesondere bevorzugt ist die Herstellung von mit Patienten autologem Endothel beschichteten Blutgefäßen, dadurch daß zuvor eine schonende Entfernung des Spenderendothels vorgenommen wird und die übrigen Wandstrukturen auf diese Weise vollkommen erhalten werden. Diese selektive Entfernung des Spenderendothels wird durchgeführt, indem mechanisch oder immunologisch (durch Komplement-vermittelte Lyse) das Spenderendothel entfernt wird. Insbesondere sind enzymatische Methoden zur Entfernung des Spenderendothels zu vermeiden:

Die klinischen Ergebnisse zeigen, daß eine klinisch relevante Abstoßungsreaktion im standardmäßig durchgeführten zytoimmunologischen Monitoring nicht gefunden wurde, wenn auf eine Vorbehandlung der Matrix, die zu einer Dezellularisierung führt, insbesondere eine enzymatische Vorbehandlung, verzichtet wird.

In einer weiteren Ausführungsform umfaßt das erfindungsgemäße Verfahren zusätzlich die Vorbeschichtung der kyropräservierten Gefäße mit Patientenautologem Serum.

Eine Vorbeschichtung mit Patienten-autologem Serum, wobei physiologische Konzentrationen eingesetzt werden, fördert nicht nur die Haftung, sondern auch die funktionelle Differenzierung der aufgesäten Endothelschicht.

Durch in vitro Studien, die zur Endothelialisierung von kryopräservierten Venen durchgeführt wurden, konnte gezeigt werden, daß eine Vorbeschichtung der Venen mit Serum eine ideale Matrix zur Zellansiedelung auf der Vene darstellt.

Diese Vorbeschichtung war einer Vorbeschichtung mit dem zur Zeit gültigen Standard der Vorbeschichtung mit Fibronectin mit und ohne einem Proteoglycan (z.B. Heparinsulfat, siehe U.S. Pat. No. 5,192,312; U.S. Pat. No. 5,632,778; U.S. Pat. No. 5,613,982, U.S. Pat. No. 5,843,182, WO 95/24873, Zilla P. et al., Endothelial cell seeding of polytetrafluoroethylene grafts in humans. J. Vasc. Surg. 6: 535-541, 1987) deutlich überlegen. Dies stellt einen bemerkenswerten Vorteil dar, da die klinische Verwendung von Fibronectin in Europa nicht zugelassen ist.

In einer besonders bevorzugten Ausführungsform wird in dem erfindungsgemäßen Verfahren die Beschichtung eines Gefäßes (bzw. eines Hohlorgans) mit Patienten-autologen Endothelzellen mit Hilfe eines speziellen Kultivationsgerätes durchgeführt, das dadurch gekennzeichnet ist, daß ein Druckgradient aufgebaut werden kann zwischen dem Lumen des Gefäßes (bzw. Hohlorgans) und dem Außenbereich des Gefäßes, der verhindert, daß die Wände des Gefäßes (bzw. des Hohlorgans) kollabieren. Außerdem wird es spezifisch ermöglicht, unerwünschte Stoffwechselprodukte der Gefäßwand durch transmurale Filtration auszuwaschen.

Das erfindungsgemäße Beschichtungsverfahren kann bei sämtlichen natürlichen Hohlorganen und deren Bestandteilen angewendet werden, beispielsweise bei natürlichen Blutgefäßen. Der Fachmann kann das erfindungsgemäße Verfahren auch anwenden zur Endothelialisierung von Blutgefäßklappen und Herzklappen, von Lymphgefäßen und ihren Klappen, von Harnleitern und Harnblase, Samenleitern, Bronchien und von jeglichem prosthetischem Ersatz solcher Hohlorgane.

Vorzugsweise wird das erfindungsgemäße Beschichtungsverfahren bei kryopräservierten Spendergefäßen (Venen oder Arterien) sowie bei allogenem Herzklappenersatz eingesetzt. Des weiteren kann das erfindungsgemäße Beschichtungsverfahren bei entsprechenden Xenografts eingesetzt werden.

In einer Ausführungsform umfaßt das erfindungsgemäße Verfahren zur Herstellung der Hohlorgane die Kryopräservierung entsprechender Hohlorgane bis zum Zeitpunkt der Verwendung, ihr Auftauen, die selektive Entfernung ihrer Epithel- bzw. Endothelauskleidung, bei der eine enzymatische Behandlung vermieden wird, die Isolierung von Patienten-autologem Epithel, vorzugsweise Endothelzellen und besonders bevorzugt vaskulären Endothelzellen, das Vorbeschichten des kryopräservierten Hohlorgans, vorzugsweise des Spendergefäßes. insbesondere einer kryopräservierten Spendervene, mit Patientenautologem Serum und die dauerhafte Beschichtung des Hohlorgans (bzw. des Spendergefäßes) mit den Patienten-autologen Epithelzellen, z.B. den Endothelzellen, besonders bevorzugt mit Hilfe des erfindungsgemäßen Kultivationsgerätes.

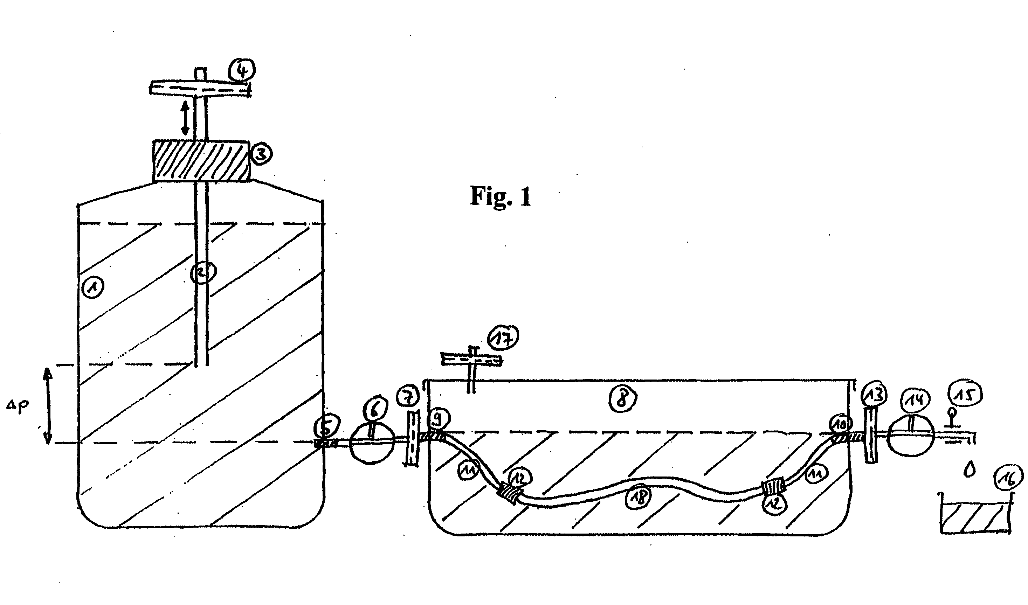

Ein besonders bevorzugtes Kultivationsgerät ist in Figur 1 gezeigt. Dieses Kultivationsgerät umfaßt ein mit Medium gefülltes Kulturgefäß, in dem sich das Gefäß befindet (z.B. eine Vene). Das Lumen der beiden Venenenden ist mittels zweier Schläuche mit den beiden Auslässen des Kulturgefäßes verbunden. Dazwischen geschaltet sind zwei Sterilfilter (7, 13) und außerhalb des Gefäßes zwei Dreiwegehähne (6, 14). Der eine Schlauch ist mit einem Medium-gefüllten Boyle-Marriott'schen Gefäß (1) verbunden. Der andere Schlauch endet an einem Abwurfgefäß. Der Druckgradient Ap (abhängig von Kanüle (2)) und die Schlauchklemme (15) werden so eingestellt, daß ein Kollabieren der Vene verhindert wird und ein konstanter Flow von Kulturmedium zur Ernährung der Endothelzellen entsteht. Durch Öffnen der Schlauchklemme (15) kann beliebig oft ein kompletter Mediumwechsel innerhalb der Vene durchgeführt werden. Der Mediumwechsel kann auch durch elektronisch gesteuerte Pumpen automatisiert werden.

Die erfindungsgemäßen Gefäße können in der Herz- und Gefäßchirurgie verwendet werden. Insbesondere lassen sie sich verwenden als aortokoronarer Bypass bei koronarer Herzkrankheit und als Gefäßtransplantat bei Gefäßrekonstruktionen jeglicher Art. Dies betrifft z. B. die periphere arterielle Verschlußkrankheit, aneurysmatische Veränderungen an Gefäßen, die einen Ersatz dieser Gefäße zur Folge haben, sowie sämtliche Wiederholungsoperationen am Herzen und an den Gefäßen. Insbesondere sind diese Gefäße das ideale Konduit für den Einsatz in infizierten Gebieten. Weitere Indikationen für den Einsatz derartiger Gefäße stellen eine Vielzahl angeborener Mißbildungen dar (beispielhaft seien jegliche Formen von Shuntoperationen erwähnt). Zusätzlich eignen sich derartige Gefäße zu grundlagenwissenschaftlichen Untersuchungen wie z. B. Arterioskleroseforschung oder Permeationstestung von Pharmaka.

Eine weitere Ausführungsform der Erfindung betrifft Gefäße, die mit Patienten-autologen Endothelzellen auf der Innenoberfläche ausgekleidet sind, wobei die Endothelzellen aus anderen Quellen (z.B. peripheres Blut, Knochenmark, Fettgewebe, gentechnisch verändertes oder hergestelltes Endothel, xenogenes und gegebenfalls gentechnisch verändertes xenogenes Endothel) gewonnen worden sind.

Patienten-autologes Epithel kann gentechnologisch hergestellt werden, so daß Epithel erhalten wird, das das Patienten-autologe Epithel in seinen Oberflächeneigenschaften bzw. immunologischen Eigenschaften imitiert.

Eine zusätzliche Ausführungsform betriffl die Verwendung von Matrices xenogenen Ursprungs für den Aufbau eines neuen Gefäßes und dessen Endothelialisierung (z. B. Zerstörung des Gewebes boviner Brustwandarterien durch z. B. forcierte Kryopräservierung dieser Gefäße bis auf die bindegewebige Grundstruktur der Gefäße und Aussaat von Endothel auf diese Gefäße).

In einer weiteren Ausführungsform ist das erfindungsgemäße Hohlorgan zusätzlich auf der Außenoberfläche von einem Mantel aus einem synthetischen (künstlich hergestellten) Material umschlossen.

Der Begriff synthetisches Material, wie hier verwendet, bedeutet jedes organische und/oder anorganische Produkt, das für solche Zwecke geeignet ist

In einer besonders bevorzugten Ausführungsform ist das erfindungsgemäße Hohlorgan zusätzlich von einem Mantel aus einem synthetischen resorbierbaren Material umschlossen.

In einer besonders bevorzugten Ausführungsform basiert der Mantel aus synthetischer Polyglyconsäure.

Die erfindungsgemäßen Hohlorgane, die zusätzlich von einem Mantel wie z. B. aus Polyglyconsäure umgeben sind, weisen den Vorteil auf, daß sie für mehrere Monate stabilisiert sind.

Eine weitere Ausführungsform betrifft die Aussaat von Endothelzellen zum Zwecke des Selbstaufbaus (tissue engeneering) eines Gefäßes auf resorbierbarem Material (z. B. Polydioxanon).

Die Figur dient der Erläuterung der Erfindung.

Figur 1 zeigt ein Kulturgerät zur Verwendung bei dem erfindungsgemäßen Verfahren bei kryopräservierten und nichtkryopräservierten Gefäßen.

Die nachfolgenden Beispiele erläutern die Erfindung und sind nicht als einschränkend aufzufassen.

### Beispiel 1: Patienten-autologe Endothelialisierung von kryopräservierten Venen

Bei den zu operierenden Patienten werden im präoperativen Verlauf ca. 500 ml Vollblut ohne gerinnungshemmende Substanzen entnommen, bei 4°C für 24 Stunden gelagert und anschließend die festen Bestandteile abzentrifugiert. Das Serum wird bis zur weiteren Verwendung tiefgekühlt. Simultan wird - falls nicht ohnehin vorhanden - eine Spendervene nach bekanntem Schema (Brockbank KGM et al., Cryopreserved Vein Transplantation. J. Cardiac Surg 7:170-176, 1992; Gelbish J. et al. Cryopreserved homologous saphenous vein: Early and late patency in coronary artery bypass surgical procedures. Ann Thorac Surg 42:70, 1986; Brockbank KGM et al. Functional analysis of cyropreserved veins. J Vasc Surg 11: 94-102, 1990) kryopräserviert.

In einer Voroperation wird bei dem Empfänger der zu beschichtenden Prothese in lokaler Anästhesie ein etwa 5 cm langes Venenstück entnommen. Die Zellisolierung und Vermehrung isolierter Endothelzellen erfolgt nach gängigen Zellkulturtechniken (Jaffe EA, Nachman RL, Becker CG, et al. Culture of human endothelial cells derived from umbilical veins. Identification by morphologic and immunologic criteria. J Clin Invest 52: 2745-56, 1973). Als Kulturmedium kann beispielsweise Medium 199 (Seromed) supplementiert mit 20% autologem Serum und 2 ng/ml rekombinanten bFGF (basic fibroblast growth factor) verwendet werden. Nach dem Erreichen einer für die Beschichtung ausreichenden Zellzahl wird die vorher kryopräservierte Vene im Wasserbad von 37°C aufgetaut. Die Spendervene wird nun vorzugsweise mittels eines Ballonkatheters (z. B. Fogartykatheter, Fa. Baxter), der durch die Vene im aufgeblasenen Zustand in Flußrichtung (cave Venenklappen!) gezogen wird, von den Resten des Spenderendothels befreit. In anderen Ansätzen kann das Endothel auch spezifisch durch Antikörper-induzierte Komplementlyse entfernt werden. Die Vene wird mit Patientenautologem Serum gefüllt und im Brutschrank im gefüllten Zustand für 12 - 24 Stunden bei 37°C inkubiert. Hierfür werden die beiden Enden der Vene mit einem durchgängigen Adapterstopfen (der eingebunden wird) versehen, der selbst wiederum durch einen wiederentfernbaren Stopfen verschlossen wird. Im Anschluß wird durch Entfernung der Stopfen das Serum abgelassen, die vorbeschichtete Vene mit einer definierten Zellzahl (80.000-120.000 Zellen/cm2 Graftoberfläche) Patienten-autologen Endothels gefüllt und durch Wiedereinführen der Stopfen verschlossen. Nun wird die Vene für mehrere Stunden in einem Rotationsgerät, basierend auf einem in der Literatur bereits mehrfach beschriebenen Rotationsgerät [Kadletz M, Moser R, Preiss P, et al. In vitro lining of fibronectin coated PTFE grafts with cryopreserved saphenous vein endothelial cells. Thorac Cardiovasc Surg, 35 Spec No 2: 143-147, 11/1987] im Brutschrank (Functionline, Heraeus Instruments) bei 37°C rotiert. Dabei kommt es zur gleichmäßigen Adhäsion der Zellen auf der Graftinnenoberfläche. Danach wird die beschichtete Vene dem Rotationsgerät entnommen und in das spezielle Kultivationsgerät (siehe Fig. 1) eingebracht.

Figur 1 zeigt das speziell für die Kultivation von endothelialisierten Gefäßen entwickelte Kultivationsgerät, das durchgängig aus biologisch inerten, autoklavierbaren Teilen besteht. Durch dieses Gerät kann ein konstanter Druckgradient (0 - 20 cm; H₂O) über die Venenwand aufgebaut werden, um ein Kollabieren der zu kultivierenden Vene zu verhindern. Des weiteren kann ein kontinuierlicher Mediumwechsel unter sterilen Kautelen durchgeführt werden.

Das Boyle Marriott'sche Gefäß (1) bestehend aus einer 500 ml Glasflasche ist mit einem für eine Kanüle (2) durchgängigen Stopfen (3) versehen. Diese Kanüle, die zur Einstellung des Druckgradienten dient, ist zur Atmosphäre hin mit einem Sterilfilter (Millipore) (4) versehen. Im unteren Drittel der Flasche befindet sich eine Öffnung (5), die mittels eines Dreiwegehahns (6) und eines weiteren Sterilfilters (7) mit dem eigentlichen Kulturgefäß (8) verbunden ist. Als Kulturgefäß dient eine verschließbare Glaskulturschale, die auf beiden Seiten auf mittleren Niveau mit einem zu- (9) und abführenden (10), d. h. die Wand des Gefäßes penetrierenden Stutzen versehen ist. Um Venen verschiedener Länge kultivieren zu können, wird jeweils ein Vitonschlauch (11) den beiden Stutzen von der Innenseite des Kulturgefäßes her aufgesetzt, die später mit einem Venenadapter (12) verbunden werden können, so daß ein kontinuierlicher Mediumfluß von der Boyle Mariott'schen Flasche zum Abwurfgefäß gewährleistet ist. An Stelle der später einzusetzenden Vene ist ein Vitonschlauch (Labokron) mit den zugehörenden Adaptern (identisch zu den bei der Vene benutzten) zwischengeschaltet. Der Auslaß ist wiederum über einen Sterilfilter (13) und nachgeschalteten Dreiwegehahn (14) und eine regulierbare Schlauchklemme (15) mit einem Abwurfgefäß (16) verbunden. Im verschlossenen Zustand erfolgt der für die Kultivation von Zellen notwendige Gasaustausch (5% CO₂, gesättigter Wasserdampf) über einen am Deckel der Schale angebrachten Sterilfilter (17). Zur Vorbereitung des Systems wird nun die Boyle Marriott'sche Flasche und das Kulturgefäß zu je zwei Dritteln mit Kulturmedium gefüllt und das Schlauchsystem durch Öffnen der Dreiwegehähne entlüftet. Die Vorbereitungen zur Kultivation der Vene sind abgeschlossen.

Zum Einbringen der beschichteten Vene (18) in das spezielle Kulturgefäß wird dieses geöffnet. Die Adapter der Vene werden nun nach Entfernen der Verschlußstopfen und des bislang für die Vene als Platzhalter dienenden Vitonschlauches mit dem Kulturgefäß verbunden. Bei diesem Vorgehen ist darauf zu achten, daß dieses Prozedere luftblasenfrei erfolgt (Diskonnektion und Konnektion unter Mediumspiegel). Der tägliche Mediumwechsel wird durch Öffnen der Schlauchklemme und Ablassen von ca. 20 ml Medium durchgeführt. Des weiteren ist es erforderlich, die Mediumspiegel der Boyle Marriot'schen Flasche und des Kulturgefäßes zu kontrollieren, um eventuelle Undichtigkeiten in der Vene rechtzeitig erkennen zu können. Nach 4-9, vozugsweise 6-9 tägiger Kultivation im Brutschrank erfolgt die Implantation des Grafts nach chirurgischen Standardmethoden (Kirklin JW, Barratt-Boyes BG: Cardiac Surgery: S 299 - 311.New York, Edinburgh, London, Madrid, Melbourne, Tokyo. 1993).

Die Verwendung des vorstehend-beschriebenen Kultivationsgerätes (Fig. 1) in dem erfindungsgemäßen Beschichtungsverfahren ist besonders bevorzugt, da sich die folgenden Vorteile feststellen ließen:

Es wird ein konstanter Druckgradient über die Venenwand erzeugt. Dadurch wird ein Kollabieren der Vene verhindert. Zusätzlich kommt es zum Transport von Medium über die Venenwand, die zur Ernährung der aufgebrachten Endothelzellen und der Venenwand selbst dient. Durch das Prinzip der Boyle-Marriot'schen Flasche wird auch bei sinkendem Mediumspiegel der Druckgradient konstant gehalten. Der für die Ernährung der sich etablierenden Endothelzellschicht (neugeschaffene Intima des Gefäßes) notwendige komplette Mediumwechsel läßt sich durch die regulierbare Schlauchklemme leicht und unter sterilen Kautelen durchführen. Dieser Vorgang kann auch durch computergesteuerte Pumpen automatisiert werden. Bei diesem Gerät handelt es sich um ein einfaches, leicht zu handhabendes, billiges und sicheres Hilfsmittel zur Endothelialisierung von Gefäßen jeder Art.

Perfusionsversuche von endothelialisierten kryopräservierten oder nichtkryopräservierten Spendergefäßen zeigten keine Unterschiede in der Endothelmorphologie und Scherkraftstabilität zu vollkommen intakten, frisch gewonnenen Venen oder Arterien.

Zur Beurteilung der Langzeitoffenheitsrate der so behandelten Gefäße auf statistisch gesichertem Signifikanzniveau fehlt es derzeit noch an einer ausreichend groß angelegten klinischen Studie. Der erste klinische Einsatz eines derartigen Bypasses im Januar 1998 verlief außerordentlich erfolgreich.

### Beispiel 2: Patienten-autologe Endothelialisierung von nicht-kryopräservierten Venen

Die Endothelialisierung von einer nicht-kryopräservierten Venen wurde entsprechend dem Verfahren des Beispiels 1 durchgeführt.

### Beispiel 3: Patienten-autologe Endothelialisierung von einem anderen Gefäß, z.B. einer Arterie

Die Endothelialisierung von einer Arterie wurde genauso durchgeführt wie die im Beispiel 1 beschriebene Endothelialisierung von einer Vene.

### Beispiel 4: Epithelialisierung von einem anderen Hohlorgan, nämlich von einem Harnleiter.

Die Epithelialisierung von einem Harnleiter wurde entsprechend der im Beispiel 1 beschriebenen Endothelialisierung von einer kryopräservierten Vene durchgeführt, mit dem Unterschied, daß statt Endothel Urothel verwendet wurde.

### Beispiel 5: Beschichtungsverfahren, wobei die Endothelzellen aus anderen Quellen (s. o.) gewonnen worden sind

Beschichtungsverfahren wie Beispiel 1. Die Isolation der entsprechenden Endothelzellen erfolgte aus peripherem Blut, Knochenmark und Bauchfett. Diese Isolierung von Endothelzellen hat für Patienten einen deutlichen Nutzen, da Verfahren auch für solche Patienten verfügbar sind, die über kein ausreichendes vaskuläres Substrat für die Gewinnung von autologem Endothel verfügen. Zusätzlich sind diese Verfahren weniger invasiv für den Patienten.

## Patentansprüche

1. Natürliches Kryopräserviertes Hohlorgan und dessen sämtliche Bestandteile, das an der Innenoberfläche bzw. der luminalen Oberfläche eine Auskleidung mit Patientenautologem Epithel aufweist.

2. Hohlorgan gemäß Anspruch 1, **dadurch gekennzeichnet, daß** das Patienten-autologe Epithel Patienten-autologe Endothelzellen sind.

3. Hohlorgan gemäß Anspruch 1 oder 2, **dadurch gekennzeichnet, daß** das Hohlorgan ein Gefäß ist.

4. Hohlorgan gemäß Anspruch 3, **dadurch gekennzeichnet, daß** das Gefäß ein allo- oder xenogen Gefäß ist.

5. Hohlorgan gemäß einem der Ansprüche 2-4, **dadurch gekennzeichnet, daß** die Patienten-autologen Endothelzellen aus peripherem Blut, Knochenmark oder Fettgewebe stammen oder aus gentechnisch verändertem oder hergestelltem Endothel oder aus gentechnisch verändertem xenogenem Endothel bestehen.

6. Hohlorgan gemäß einem der Ansprüche 1-5, **dadurch gekennzeichnet, daß** das Hohlorgan zusätzlich von einem Mantel aus einem synthetischen Material umschlossen ist.

7. Hohlorgan gemäß Anspruch 6, **dadurch gekennzeichnet, daß** das Hohlorgan von einem Mantel aus einem synthetischen resorbierbaren Material umschlossen ist.

8. Hohlorgan gemäß Anspruch 7, **dadurch gekennzeichnet, daß** der Mantel synthetischen resorbierbaren Material aus Polyglyconsäure besteht.

9. Verfahren zur Herstellung der Hohlorgane gemäß einem der Ansprüche 1 bis 5, umfassend die Beschichtung von einem kyropräservierten Spenderhohlorgan mit Patienten-autologem Epithel.

10. Verfahren zur Herstellung der Gefäße gemäß Anspruch 3 oder 4, umfassend die Beschichtung von einem kyropräservierten Spendergefäß mit Patienten-autologen Endothelzellen.

11. Verfahren gemäß Anspruch 10, **dadurch gekennzeichnet, daß** vor der Beschichtung mit Patienten-autologen Endothelzellen eine selektive Entfernung des Spenderendothels vorgenommen wird.

12. Verfahren gemäß Anspruch 11, **dadurch gekennzeichnet, daß** die selektive Entfernung des Spenderendothels mechanisch, immunologisch (durch Komplement - vermittelte Lyse) vorgenommen wird.

13. Verfahren gemäß einem der Ansprüche 10-12, **dadurch gekennzeichnet, daß** vor der Beschichtung eine Vorbeschichtung der kryopräservierten Gefäße mit Patienten-autologem Serum durchgeführt wird.

14. Verfahren nach einem der Ansprüche 9-13, **dadurch gekennzeichnet, daß** die Beschichtung mit einem Kultivationsgerät durchgeführt wird, mit dem ein Druckgradient aufgebaut werden kann zwischen dem Lumen des Hohlorgans und dem Außenbereich des Hohlorgans, der verhindert, daß die Wände des Hohlorgans kollabieren.

15. Verfahren nach einem der Ansprüche 9-13, **dadurch gekennzeichnet, daß** die Beschichtung mit einem Kultivationsgerät durchgeführt wird, umfassend ein Boyle-Mariott'sches Gefäß (1), eine Kanüle (2) mit durchgängigen Stopfen (3), eine Öffnung (5) und ein Kulturgefäß (8), das auf beiden Seiten mit einem zu- (9) und abführenden (10) Stutzen versehen ist.

16. Verwendung des Hohlorgans gemäß einem der Ansprüche 1 bis 8 zur Herstellung eines mittels zur Verwendung in der Herz- und Gefäßchirurgie, der visceralen Chirurgie und der Urologie.

17. Verwendung des Hohlorgans gemäß einem der Ansprüche 1 bis 8 zur Herstellung eines mittels zur Verwendung als aortokoronarer Bypass bei koronarer Herzkrankheit und als Gefäßtransplantat.

18. Verwendung des Hohlorgans gemäß einem der Ansprüche 1 bis 8 zur Herstellung eines mittels zur Verwendung bei peripherer arterieller Verschlußkrankheit, aneurysmatischer Veränderung an Gefäßen und bei angeborenen Mißbildungen von Gefäßen.

19. Kultivationsgefäß geeignet fur die Beschichtung der Hohlorgane gemäß einem der Ansprüche 1 bis 8 umfassend ein Boyle-Mariott'sches Gefäß (1), eine Kanüle (2) mit durchgängigen Stopfen (3), eine Öffnung (5) und ein Kulturgefäß (8), das auf beiden Seiten mit einem zu- (9) und abführenden (10) Stutzen versehen ist.

20. Transplantat umfassend ein Hohlorgan gemäß einem der Ansprüche 1 bis 8.

## Claims

1. Natural cryopreserved hollow organ and its entire components, which on the inner surface respectively the luminal surface is lined with patient autologous epithelium.

2. Hollow organ according to claim 1, wherein the patient autologous epithelium is patient autologous endothelial cells.

3. Hollow organ according to claim 1 or 2, wherein the hollow organ is a vessel.

4. Hollow organ according to claim 3, wherein the vessel is an allogeneic or xenogeneic vessel.

5. Hollow organ according to any one of claims 2 to 4, wherein the patient autologous endothelial cells are derived from peripheral blood, bone marrow or adipose tissue or consist of genetically modified or produced endothelium or genetically modified xenogeneic endothelium.

6. Hollow organ according to any one of claims 1 to 5, wherein the hollow organ is additionally enclosed by a covering of a synthetic material.

7. Hollow organ according to claim 6, wherein the hollow organ is enclosed by a covering of a synthetic absorbable material.

8. Hollow organ according to claim 7, wherein the covering of synthetic absorbable material consists of poly-gluconic acid.

9. Method for preparing the hollow organs according to any one of claims 1 to 5, comprising coating a cryopreserved donor hollow organ with patient autologous epithelium.

10. Method of preparing the vessels according to claim 3 or 4, comprising coating a cryopreserved donor vessel with patient autologous endothelial cells.

11. Method according to claim 10, wherein prior to coating with patient autologous endothelial cells the donor endothelium is selectively removed.

12. Method according to claim 11, wherein the selective removal of donor endothelium is by mechanical, immunological (via complement-mediated lysis) means.

13. Method according to any one of claims 10 to 12, wherein prior to coating the cryopreserved vessels are pre-coated with patient autologous serum.

14. Method according to any one of claims 9 to 13, wherein coating is effected by a cultivation device with which a pressure gradient can be established between the lumen of the hollow organ and the exterior of the hollow organ, which prevents the walls of the hollow organ from collapsing.

15. Method according to any one of claims 9 to 13, wherein the coating is effected by a cultivation device comprising a Boyle-Mariott's vessel (1), a cannula (2) with penetrable stoppers (3), an opening (5) and a culture vessel (8) which on both of its sides has an inlet (9) and outlet (10) fitting.

16. Use of the hollow organ according to any one of claims 1 to 8 for the preparation of a means for use in cardiac and vessel surgery, visceral surgery and urology.

17. Use of the hollow organ according to any one of claims 1 to 8 for the preparation of a means for use as aortocoronary bypass in case of coronary heart disease and as vascular graft.

18. Use of the hollow organ according to any one of claims 1 to 8 for the preparation of a means for use in case of peripheral arterial occlusive disease, aneurysmatic changes of vessels and innate malformations of vessels.

19. Cultivation vessel suitable for use in coating the hollow organs according to any one of claims 1 to 8 comprising a Boyle-Mariott's vessel (1), a cannula (2) with penetrable stoppers (3), an opening (5) and a culture vessel (8) which on both of its sides has an inlet (9) and outlet (10) fitting.

20. Transplant comprising a hollow organ according to any one of claims 1 to 8.

## Revendications

1. Organe creux naturel cryopréservé et ensemble de ses éléments, qui présente sur sa surface interne ou la surface interne luminale un revêtement avec de l'épithélium autologue du patient.

2. Organe creux selon la revendication 1, **caractérisé en ce que** l'épithélium autologue du patient sont des cellules endothéliales autologues du patient.

3. Organe creux selon la revendication 1 ou 2, **caractérisé en ce que** l'organe creux est un vaisseau.

4. Organe creux selon la revendication 3, **caractérisé en ce que** le vaisseau est un vaisseau allo- ou xénogène.

5. Organe creux selon l'une des revendications 2-4, **caractérisé en ce que** les cellules endothéliales autologues du patient proviennent du sang périphérique, de moelle osseuse ou de tissus adipeux ou consistent en de l'endothélium modifié ou préparé par génie génétique ou en de l'endothélium xénogène modifié par génie génétique.

6. Organe creux selon l'une des revendications 1-5, **caractérisé en ce que** l'organe creux est en outre entouré par une enveloppe en matériau synthétique.

7. Organe creux selon la revendication 6, **caractérisé en ce que** l'organe creux est entouré par une enveloppe en matériau synthétique résorbable.

8. Organe creux selon la revendication 7, **caractérisé en ce que** l'enveloppe de matériau synthétique résorbable consiste en de l'acide polygluconique.

9. Procédé pour la préparation d'organes creux selon l'une des revendications 1 à 5, comportant le revêtement d'un organe creux dispensateur cryopréservé avec de l'épithélium autologue des patients.

10. Procédé pour la préparation de vaisseaux selon la revendication 3 ou 4, comportant le revêtement d'un vaisseau dispensateur cryopréservé avec des cellules endothéliales autologues des patients.

11. Procédé selon la revendication 10, **caractérisé en ce que**, avant le revêtement avec des cellules endothéliales autologues des patients, on effectue une élimination sélective de l'endothélium du dispensateur.

12. Procédé selon la revendication 11, **caractérisé en ce que** l'élimination sélective de l'endothélium du dispensateur est effectuée mécaniquement, immunologiquement (par une lyse avec médiation par du complément).

13. Procédé selon l'une des revendications 10-12, **caractérisé en ce que**, avant le revêtement, on effectue un pré-revêtement du vaisseau cryopréservé avec du sérum autologue des patients.

14. Procédé selon l'une des revendications 9-13, **caractérisé en ce qu'**on effectue le revêtement avec un appareil de culture, avec lequel un gradient de pression peut être établi entre la lumière de l'organe creux et la partie extérieure de l'organe creux, qui empêche le collapse des parois de l'organe creux.

15. Procédé selon l'une des revendications 9-13, **caractérisé en ce que** le revêtement est effectué avec un appareil de culture comportant un récipient de Boyle-Mariott (1), une canule (2) avec bouchon perforé (3), une ouverture (5) et un récipient de culture (8), qui est muni sur les deux côtés d'une tubulure d'entrée (9) et de sortie (10).

16. Utilisation de l'organe creux selon l'une des revendications 1 à 8 pour la préparation d'un moyen pour utilisation en chirurgie cardiaque et vasculaire, en chirurgie viscérale et en urologie.

17. Utilisation de l'organe creux selon l'une des revendications 1 à 8 pour la préparation d'un moyen pour utilisation pour le pontage aorto-coronarien dans une affection cardiaque coronaire et comme transplant de vaisseaux.

18. Utilisation de l'organe creux selon l'une des revendications 1 à 8 pour la préparation d'un moyen pour utilisation dans une affection obstruant les artères périphériques, dans l'altération anévrismatique des vaisseaux et dans les malformations congénitales des vaisseaux.

19. Récipient de culture approprié pour le revêtement des organes creux selon l'une des revendications 1 à 8, comportant un récipient de Boyle-Mariott (1), une canule (2) avec bouchon perforé (3), une ouverture (5) et un récipient de culture (8), qui est muni sur les deux côtés d'une tubulure d'entrée (9) et de sortie (10).

20. Transplant comportant un organe creux selon l'une des revendications 1 à 8.
